# FASCICULE DE BREVET EUROPEEN

(11) **EP 0 498 741 B1**
(45) Date de publication et mention de la délivrance du brevet: **05.07.1995**
(21) Numéro de dépôt: 92420011.6
(22) Date de dépôt: 10.01.1992
(51) Int. Cl.: A61M 1/30

(54) **Procédé de commande de la circulation du sang dans un circuit à aiguille unique**
Verfahren zum Steuern der Blutzirkulation in einem Einnadelkreislauf
Blood circulation command process in a single needle circuit

(30) Priorité: 06.02.1991 FR 9101566
(43) Date de publication de la demande: 12.08.1992
(73) Titulaire: HOSPAL INDUSTRIE, F-69883 Meyzieu Cédex (FR)
(72) Inventeur: Riquier, Jean-Claude, F-69140 Rilleux (FR); Chevallet, Jacques, F-69360 Serezin du Rhone (FR)

(56) Documents cités:
- EP-A- 0 104 896
- FR-A- 2 632 360
- GB-A- 2 141 936
- US-A- 4 643 714
- US-E- 29 346

## Description

La présente invention concerne le domaine de la circulation extracorporelle du sang dans un circuit à aiguille unique. Plus particulièrement, l'invention se rapporte à un procédé de commande de l'alternance des phases artérielles ou phases d'aspiration du sang et des phases veineuses ou phases de restitution du sang.

La mise en circulation extracorporelle du sang en vue de son traitement pose des problèmes. En effet, le sang doit tout d'abord être prélevé du patient, traité par un dispositif approprié puis restitué au patient. Le prélèvement et la restitution au patient peuvent être effectués par deux accès différents. Cependant, lorsqu'il s'agit de traitements devant être effectués de façon répétitive, tels que les traitements par dialyse chez les insuffisants rénaux chroniques, il est intéressant de limiter le nombre de piqûres et d'utiliser un circuit extracorporel à aiguille unique dans lequel le sang est prélevé et restitué au patient par un accès vasculaire unique. Dans un tel cas, le sang devra circuler dans l'aiguille alternativement dans un sens puis dans l'autre. Il faut donc résoudre le problème de l'alternance des phases d'aspiration ou phases artérielles et des phases de restitution ou phases veineuses.

Le document constitué par la demande FR 2 548 906 a trait à un circuit extracorporel à aiguille unique comprenant une portion artérielle comportant un réservoir artériel et des moyens d'ouverture et de fermeture du circuit situés en amont du réservoir artériel et en aval de l'aiguille ainsi qu'une portion veineuse comportant un réservoir veineux et des moyens d'ouverture et de fermeture du circuit situés en aval du réservoir veineux et en amont de l'aiguille.

Selon ce document, on commande la mise en oeuvre de la phase veineuse ou phase de restitution du sang en réponse à la présence d'une quantité limite haute de liquide à l'intérieur du réservoir veineux et on commande la mise en oeuvre de la phase artérielle ou phase d'aspiration du sang en réponse à la présence d'une quantité limite basse de liquide dans le réservoir artériel.

Selon l'auteur de ce document, un tel procédé de commande de mise en oeuvre alternativement des phases artérielles et veineuses est autorégulé et permet une utilisation optimale du temps de traitement car la phase artérielle n'est déclenchée que lorsque la quantité de liquide présente dans le réservoir artériel devient insuffisante et la phase veineuse n'est déclenchée que lorsqu'une quantité adéquate de liquide est présente dans le réservoir veineux.

Cependant, un tel procédé de commande de l'alternance des phases artérielles et veineuses n'est pas adapté à la réalisation des conditions de sécurité optimales. En effet, lors de la phase artérielle, la quantité de liquide augmente simultanément dans les deux réservoirs artériel et veineux. Or, la présence d'une quantité limite haute n'est détectée que dans le réservoir veineux ; il est donc possible qu'une quantité trop importante de liquide soit présente dans le réservoir artériel avant que la quantité limite haute soit atteinte dans le réservoir veineux, ce qui conduit alors à des débordements de sang hors du réservoir artériel.

De même, lors de la phase veineuse, la quantité de liquide diminue simultanément dans les deux réservoirs ; or seule la présence d'une quantité limite basse dans le réservoir artériel est détectée. Ceci peut conduire à ce que la quantité de liquide présente dans le réservoir veineux devienne insuffisante. On risque alors de restituer au patient non plus seulement du sang traité, mais également de l'air, ce qui peut entraîner des troubles graves tels qu'une embolie.

Afin de pallier ces inconvénients et de proposer un procédé présentant des conditions de sécurité améliorées, la présente invention a pour objet un procédé de commande de l'alternance des phases d'aspiration et de restitution du sang dans un circuit extracorporel à aiguille unique consistant en une portion artérielle comportant un réservoir artériel ainsi que des moyens d'ouverture et de fermeture du circuit situés en amont dudit réservoir et une portion veineuse comportant un réservoir veineux ainsi que des moyens d'ouverture et de fermeture du circuit situés en aval dudit réservoir,
le procédé consistant à commander la succession de la phase d'aspiration à la phase de restitution lorsqu'une valeur prédéterminée indicative d'un niveau de remplissage bas est mesurée dans un réservoir, et à commander la succession de la phase de restitution à la phase d'aspiration lorsqu'une valeur prédéterminée indicative d'un niveau de remplissage haut est mesurée dans un réservoir,
caractérisé en ce que la succession de la phase d'aspiration à la phase de restitution est commandée lorsque la valeur prédéterminée indicative d'un niveau de remplissage bas est mesurée dans le réservoir où ce niveau de remplissage bas est atteint en premier et en ce que la succession de la phase de restitution à la phase d'aspiration est commandée lorsque la valeur prédéterminée indicative d'un niveau de remplissage haut est mesurée dans le réservoir où ce niveau de remplissage haut est atteint en premier.

Selon un mode de réalisation particulier, le procédé objet de la présente invention consiste à repérer la présence d'une quantité maximale déterminée de liquide dans les réservoirs en détectant l'atteinte d'un niveau haut de liquide dans lesdits réservoirs et à repérer la présence d'une quantité minimale déterminée de liquide dans les réservoirs en détectant l'atteinte d'un niveau bas de liquide dans lesdits réservoirs.

Ainsi, le contrôle des quantités de liquide à l'intérieur des chambres est obtenu directement sans qu'il soit nécessaire d'effectuer de corrélation telle que la corrélation entre la quantité de liquide présente dans une chambre et la pression existant dans cette chambre.

En outre, les risques de débordement de réservoir ou d'introduction d'air dans le sang en circulation sont totalement évités. De plus, le procédé de commande est ainsi particulièrement adapté aux dispositifs dans lesquels les pressions artérielles et veineuses sont maintenues sensiblement constantes.

Selon un mode de réalisation particulièrement avantageux, le procédé selon l'invention consiste en outre à contrôler que la quantité maximale déterminée de liquide est obtenue simultanément dans les deux réservoirs et que la quantité minimale déterminée de liquide est obtenue simultanément dans les deux réservoirs.

Ceci permet de maîtriser totalement le volume de sang disponible pour le traitement, et d'obtenir ainsi une optimisation de l'efficacité du traitement pour une durée déterminée.

D'autres caractéristiques et avantages de la présente invention apparaîtront au cours de la description plus détaillée qui va suivre, en référence aux dessins qui illustrent de façon schématique différents modes de réalisation de l'invention.

La description se réfère à un traitement extracorporel du sang au moyen d'un appareil de dialyse et/ou d'ultrafiltration. Cependant, le procédé selon l'invention peut être utilisé pour toute circulation extracorporelle de sang dans un circuit à aiguille unique.

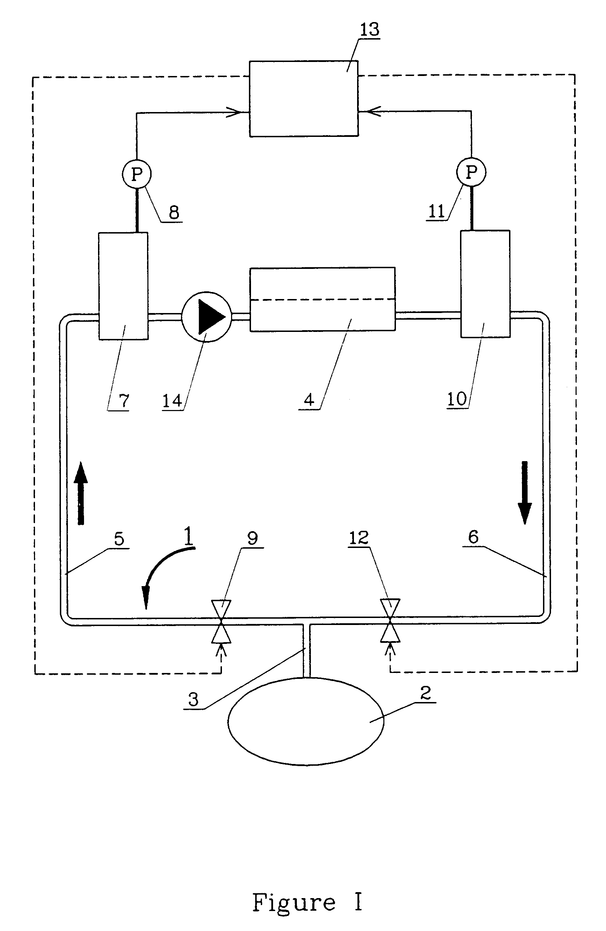

La figure 1 illustre un premier mode de réalisation de la présente invention.

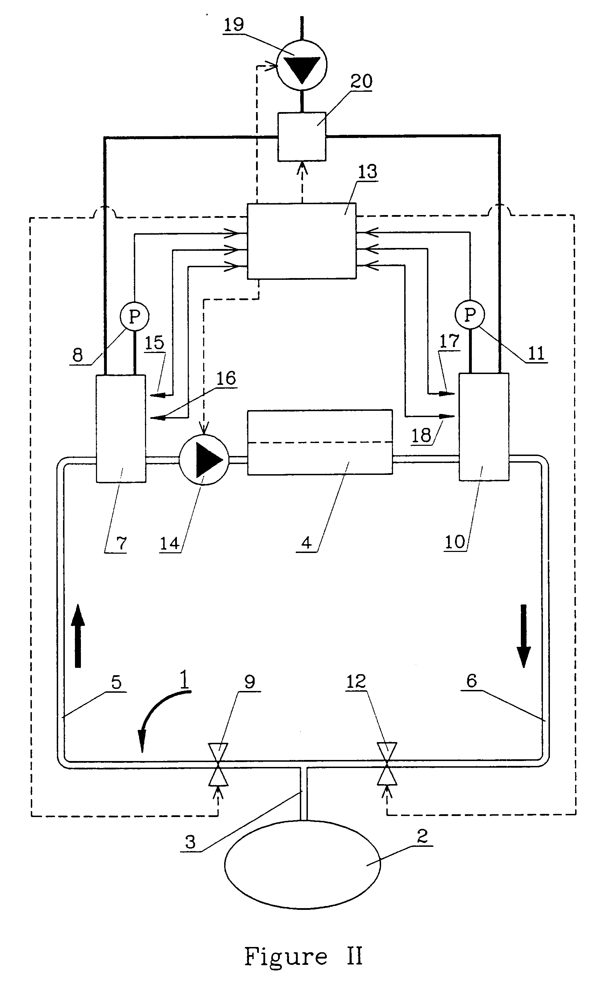

La figure 2 illustre un second mode de réalisation de la présente invention.

Selon le mode de réalisation représenté sur la figure I, le circuit extracorporel 1 permet de mettre en circulation le sang prélevé à un patient 2 par un accès unique 3 et restitué au patient après traitement par un dispositif tel qu'un hémodialyseur 4. Le circuit extracorporel 1 comporte une portion artérielle 5 destinée à la circulation du sang à traiter entre l'aiguille 3 et l'hémodialyseur 4 et une portion veineuse 6 destinée à la circulation du sang traité entre l'hémodialyseur 4 et l'aiguille 3.

La portion artérielle 5 comporte une réservoir artériel 7 muni d'un capteur de pression 8 ainsi que des moyens d'ouverture et de fermeture du circuit tels qu'un clamp 9. De même, la portion veineuse 6 comporte un réservoir veineux 10 muni d'un capteur de pression 11 ainsi que des moyens d'ouverture et de fermeture du circuit tels qu'un clamp 12. Les informations provenant des capteurs de pression 8 et 11 sont transmises à une unité 13 qui commande l'ouverture et la fermeture des clamps 9 et 12.

En outre, une pompe 14 est située sur la portion artérielle 5 en aval du réservoir 7 et en amont de l'hémodialyseur 4.

Le fonctionnement d'un tel circuit extracorporel à aiguille unique s'effectue, de façon classique, par alternance de phases artérielles et de phases veineuses.

Durant la phase artérielle, le clamp 9 est ouvert et le clamp 12 fermé ; le sang est prélevé du patient et circule, grâce notamment à l'action de la pompe 14, à travers l'aiguille 3, la portion artérielle 5, l'hémodialyseur 4 et la portion veineuse 6 ; étant donné que le clamp 12 est fermé, la quantité de liquide à l'intérieur des réservoirs 7 et 10 augmente. Ces réservoirs étant fermés, lorsque le volume de liquide augmente, leur pression interne augmente aussi.

Par contre, durant la phase veineuse, le clamp 12 est ouvert et le clamp 9 est fermé ; le sang est restitué au patient ; la circulation du sang au travers de l'hémodialyseur 4 est maintenue grâce au liquide présent dans le réservoir 7. Cependant, la quantité de liquide à l'intérieur des réservoirs 7 et 10 diminue et leur pression interne diminue aussi. La pression existant à l'intérieur de la portion artérielle est toujours négative et varie entre environ - 200 mmHg (-2,67.10⁴Pa par rapport à la pression atmosphérique) à la fin de la phase artérielle et - 350 mmHg (-4,6.10⁴Pa par rapport à la pression atmosphérique) à la fin de la phase veineuse. La pression existant à l'intérieur de la portion veineuse est une pression positive qui varie entre environ + 200 mmHg (2,67.10⁴Pa) à la fin de la phase veineuse et + 350 mmHg (4,6.10⁴Pa) à la fin de la phase artérielle.

Le réglage des pressions à l'intérieur des chambres est effectué en début de séance. Les valeurs maximales et minimales des pressions sont choisies en fonction de la géométrie connue du circuit extracorporel et notamment du volume des réservoirs 7 et 10 pour correspondre à des quantités maximales et minimales déterminées et donc à une variation déterminée de la quantité de liquide à l'intérieur de chaque réservoir entre la phase artérielle et la phase veineuse. En outre, les valeurs de pression sont choisies pour être hémocompatibles et correspondre à des niveaux appropriés de liquide à l'intérieur des réservoirs.

Selon l'invention, les mesures effectuées par les capteurs de pression 8 et 11 sont transmises à l'unité 13 qui les compare avec les valeurs mises en mémoire lors du réglage initial, par exemple - 350 mmHg et - 200 mmHg pour les valeurs de pression artérielle mesurées par le capteur 8 et + 200 mmHg et + 350 mmHg pour les valeurs de pression veineuse mesurées par le capteur 11.

Ainsi, lorsque le'dispositif est en phase artérielle et que la quantité de liquide augmente à l'intérieur des réservoirs, dès que la pression mesurée atteint - 200 mmHg dans le réservoir artériel 7 ou + 350 mmHg dans le réservoir veineux 10, l'unité 13 commande la fermeture du clamp 9 et l'ouverture du clamp 12. La phase veineuse est alors mise en oeuvre, pendant laquelle la quantité de liquide diminue à l'intérieur des réservoirs ; dans ce cas, dès que la pression mesurée atteint - 350 mmHg dans le réservoir artériel ou + 200 mmHg dans le réservoir veineux, l'unité 13 provoque le passage à la phase artérielle en commandant la fermeture du clamp 12 et l'ouverture du clamp 9.

Grâce à ce procédé de commande de l'alternance des phases artérielles et veineuses, le fonctionnement du circuit extracorporel 1 s'effectue dans des conditions de sécurité améliorées par rapport à l'art antérieur.

La figure II représente un second mode de réalisation de la présente invention. Les éléments identiques à ceux de la figure I portent les mêmes numéros que précédemment. Selon ce mode de réalisation, les réservoirs artériel 7 et veineux 10 sont munis chacun de deux détecteurs de niveau dont les positions correspondent aux quantités de liquide maximales et minimales souhaitées à l'intérieur des réservoirs. Ainsi, le réservoir artériel 7 est muni d'un détecteur de niveau haut 15 et d'un détecteur de niveau bas 16. De même, le réservoir veineux 10 est muni d'un détecteur de niveau haut 17 et d'un détecteur de niveau bas 18.

Les informations provenant des détecteurs de niveau 15, 16, 17 et 18 sont transmises à l'unité 13. Le circuit représenté ici comporte en outre une pompe à air 19 qui peut, selon la position d'un distributeur 20 communiquer avec le réservoir artériel 7 ou le réservoir veineux 10. Cette pompe est pilotée par l'unité 13 et permet d'ajouter ou de retirer de l'air des réservoirs. Un distributeur 20, dont la position est également commandée par l'unité 13 permet de mettre la pompe 19 en communication sélectivement avec le réservoir 7 ou le réservoir 10.

Selon ce mode de réalisation, dès que, en fin de phase artérielle, le liquide atteint la quantité maximale déterminée dans l'un des réservoirs, c'est-à-dire dès que l'un des détecteurs de niveau haut 15 ou 17 est atteint, il émet un signal qui est transmis à l'unité 13 qui assure alors la mise en oeuvre de la phase veineuse en commandant l'ouverture du clamp 12 et la fermeture du clamp 9. De même, en fin de phase veineuse, dès que l'un des détecteurs de niveau bas 16 ou 18 est atteint, l'unité 13 déclenche la mise en oeuvre de la phase artérielle en commandant l'ouverture du clamp 9 et la fermeture du clamp 12.

Ainsi, le changement d'une phase à l'autre ne s'effectue pas seulement en fonction de ce qui se passe dans une seule portion du circuit sans se soucier de ce qui se passe simultanément dans l'autre, mais est au contraire régulé en fonction des conditions de fonctionnement de l'ensemble du circuit. En outre, pour contrôler le niveau de liquide dans les réservoirs, il est plus simple et beaucoup plus sûr d'utiliser directement des informations provenant de capteurs de niveau plutôt que de capteurs de pression.

Afin d'optimiser l'efficacité du traitement du volume de sang en circulation extracorporelle, le procédé de l'invention permet, en outre, avantageusement de contrôler que le niveau de liquide dans chaque réservoir varie réellement entre le niveau haut et le niveau bas. En effet, à la fin de la phase artérielle, lorsque l'unité 13 est avertie que le liquide a atteint le capteur de niveau haut dans un premier réservoir, elle déclenche une mesure du temps sur le second réservoir, c'est-à-dire une mesure du temps que va mettre le liquide à atteindre le capteur de niveau bas dans ce second réservoir. La durée ainsi mesurée est ensuite comparée par l'unité 13 à une durée de consigne qui est déterminée à partir du volume connu existant entre le détecteur de niveau haut et le détecteur de niveau bas et des conditions de fonctionnement du circuit (soient le débit de la pompe 14 et les valeurs de pression réglées au début de la séance). Cette durée de consigne correspond au temps que devrait mettre le niveau de liquide à descendre s'il avait atteint le niveau haut en même temps que le liquide dans le premier réservoir. La différence entre la durée mesurée et la durée de consigne permet d'apprécier le niveau auquel se trouvait le liquide dans le second réservoir au moment où le liquide atteignait le capteur de niveau haut dans le premier réservoir.

Dans le cas où la durée mesurée ne correspond pas à la durée de consigne ou du moins n'est pas comprise dans une plage de consigne, cela signifie que les conditions de pression à l'intérieur d'au moins un réservoir ne sont pas celles souhaitées. Grâce aux mesures effectuées par les capteurs 8 et 11, il est possible à l'unité 13 de déterminer dans quel réservoir a eu lieu la dérive. On peut alors effectuer une correction en ajoutant ou en retirant de l'air au moyen de la pompe à air 19. L'efficacité de la correction peut être vérifiée en effectuant une mesure au cycle suivant. Il est naturellement possible, de façon similaire, de démarrer la mesure du temps lorsque, en fin de phase veineuse, le niveau de liquide atteint le capteur de niveau bas dans un réservoir et de mesurer le temps que met le liquide à remonter dans l'autre réservoir.

Selon l'invention, il est possible également de faire varier le volume de sang disponible au traitement en variant le positionnement des détecteurs de niveau.

La présente invention n'est pas limitée aux exemples décrits mais est susceptible de nombreuses variantes. Il est notamment possible au lieu d'utiliser, pour chaque réservoir deux détecteurs de niveau, tout ou rien, de n'utiliser qu'un seul capteur transmettant un signal directement proportionnel au niveau de liquide dans le réservoir. Ainsi, pour contrôler que le liquide atteint simultanément le niveau haut et le niveau bas dans chaque réservoir, il suffit, lorsqu'un niveau limite (haut ou bas) est atteint dans un réservoir, que l'unité 13 vérifie que le signal transmis par le capteur de niveau dans l'autre réservoir est bien compris à l'intérieur d'une plage de consigne. Dans le cas contraire, l'unité 13 commande la mise en oeuvre de la pompe 19 et du distributeur 20 afin de rétablir les conditions de fonctionnement souhaitées.

## Revendications

1. Procédé de commande de l'alternance des phases d'aspiration et de restitution du sang dans un circuit extracorporel à aiguille unique (3) consistant en une portion artérielle (5) comportant un réservoir artériel (7) ainsi que des moyens (9) d'ouverture et de fermeture du circuit situés en amont dudit réservoir (7) et une portion veineuse (6) comportant un réservoir veineux (10) ainsi que des moyens (12) d'ouverture et de fermeture du circuit situés en aval dudit réservoir (7),
le procédé consistant à commander la succession de la phase d'aspiration à la phase de restitution lorsqu'une valeur prédéterminée indicative d'un niveau de remplissage bas est mesurée dans un réservoir (7, 10), et à commander la succession de la phase de restitution à la phase d'aspiration lorsqu'une valeur prédéterminée indicative d'un niveau de remplissage haut est mesurée dans un réservoir (7, 10),
caractérisé en ce que la succession de la phase d'aspiration à la phase de restitution est commandée lorsque la valeur prédéterminée indicative d'un niveau de remplissage bas est mesurée dans le réservoir (7, 10) où ce niveau de remplissage bas est atteint en premier et en ce que la succession de la phase de restitution à la phase d'aspiration est commandée lorsque la valeur prédéterminée indicative d'un niveau de remplissage haut est mesurée dans le réservoir (7, 10) où ce niveau de remplissage haut est atteint en premier.

2. Procédé selon la revendication 1, caractérisé en ce qu'il consiste à repérer la présence d'une quantité maximale déterminée de liquide dans les réservoirs en détectant l'atteinte d'un niveau haut de liquide dans lesdits réservoirs et en ce qu'il consiste à repérer la présence d'une quantité minimale déterminée de liquide dans les réservoirs en détectant l'atteinte d'un niveau bas de liquide dans lesdits réservoirs.

3. Procédé selon l'une des revendications précédentes, caractérisé en ce qu'il consiste en outre à contrôler que la quantité maximale déterminée de liquide est obtenue simultanément dans les deux réservoirs et que la quantité minimale déterminée de liquide est obtenue simultanément dans les deux réservoirs.

4. Procédé selon la revendication 3, caractérisé en ce qu'il consiste à déterminer la quantité de liquide présente dans le réservoir veineux (respectivement artériel) lorsque la quantité maximale déterminée de liquide est obtenue dans le réservoir artériel (respectivement veineux) et à modifier la quantité d'air existant à l'intérieur d'au moins un réservoir si la quantité maximale déterminée de liquide n'est pas obtenue simultanément dans les deux réservoirs.

5. Procédé selon l'une des revendications 3 ou 4, caractérisé en ce qu'il consiste à déterminer la quantité de liquide présente dans le réservoir veineux (respectivement artériel) lorsque la quantité minimale déterminée de liquide est obtenue dans le réservoir artériel (respectivement veineux) et à modifier la quantité d'air existant à l'intérieur d'au moins un réservoir si la quantité minimale déterminée de liquide n'est pas obtenue simultanément dans les deux réservoirs.

## Claims

1. A method for controlling the alternation of the blood aspiration and return stages in an extracorporeal circuit with a single needle (3), constituted by an arterial portion (5) comprising an arterial reservoir (7), as well as means (9) for the opening and closing of the circuit situated upstream of the said reservoir (7) and a venous portion (6) comprising a venous reservoir (10), as well as means (12) for the opening and closing of the circuit situated downstream of the said reservoir (7), the method consisting in controlling the succession of the aspiration stage to the return stage when a predetermined value indicative of a low level filling is measured in a reservoir (7, 10), and in controlling the succession of the return stage to the aspiration stage when a predetermined value indicative of a high level filling is measured in a reservoir (7, 10)
characterized in that the succession of the aspiration stage to the return stage is controlled when the predetermined value indicative of a low level filling is measured in the reservoir (7, 10) where this low level is reached first, and in that the succession of the return stage to the aspiration stage is controlled when the predetermined value indicative of a high level filling is measured in the reservoir (7, 10) where this high level is reached first.

2. A method according to claim 1, characterized in that it lies in registering the presence of a specified maximum quantity of liquid in the reservoirs by detecting that a high liquid level has been reached in the said reservoir and in that it lies in registering the presence of a specified minimum quantity of liquid in the reservoirs by detecting that a low liquid level has been reached in the said reservoirs.

3. A method according to one of the preceding claims, characterized in that it lies, moreover, in checking that the specified maximum quantity of liquid is obtained simultaneously in the two reservoirs and that the specified minimum quantity of liquid is obtained simultaneously in the two reservoirs.

4. A method according to claim 3, characterized in that it lies in determining the quantity of liquid present in the venous reservoir (or arterial reservoir respectively) when the specified maximum quantity of liquid is obtained in the arterial reservoir (or venous reservoir respectively) and in modifying the quantity of air inside at least one reservoir if the specified maximum quantity of liquid is not obtained simultaneously in the two reservoirs.

5. A method according to one of claims 3 or 4, characterized in that it lies in determining the quantity of liquid present in the venous reservoir (or arterial reservoir respectively) when the specified minimum quantity of liquid is obtained in the arterial reservoir (or venous reservoir respectively) and in modifying the quantity of air inside at least one reservoir if the specified minimum quantity of liquid is not obtained simultaneously in the two reservoirs.

## Patentansprüche

1. Verfahren zur Steuerung der Wechselfolge von Absaug- und Rückführphasen von Blut in einem extrakorporellen Blutkreislauf mit einer einzigen Nadel (3), der aus einem Arterienabschnitt (5) besteht, der einen Arterienvorratsbehälter (7) umfaßt, sowie eine Öffnungs- und Schließeinrichtung (9) für den Kreislauf, die stromaufwärts von diesem Vorratsbehälter (7) angeordnet ist, und einen Venenabschnitt (6), der einen Venenvorratsbehälter (10) ebenso umfaßt, wie eine Öffnungs- und Schließeinrichtung (12) für den Kreislauf, die stromabwärts von diesem Vorratsbehälter (7) angeordnet ist, wobei das Verfahren darin besteht, die Abfolge der Saugphase auf die Rückführphase zu steuern, wenn ein vorbestimmter Wert, der ein unteres Füllniveau anzeigt, in einem Vorratsbehälter (7, 10) gemessen wird, und die Abfolge der Rückführphase auf die Saugphase zu steuern, wenn ein vorbestimmter Wert, der ein oberes Füllniveau anzeigt, in einem Vorratsbehälter (7, 10) gemessen wird, dadurch gekennzeichnet, daß die Abfolge der Saugphase auf die Rückführphase befohlen wird, wenn der vorbestimmte Wert, der ein unteres Füllniveau anzeigt, in dem Vorratsbehälter (7, 10) gemessen wird, indem dieses untere Füllniveau zuerst erreicht wird, und daß die Abfolge der Rückführphase auf die Saugphase befohlen wird, wenn der vorbestimmte Wert, der ein oberes Füllniveau anzeigt, in dem Vorratsbehälter (7, 10) gemessen wird, in dem das obere Füllniveau als erstes erreicht wird.

2. Verfahren nach Anspruch 1, dadurch gekennzeichnet, daß es darin besteht, das Vorhandensein einer festgelegten maximalen Flüssigkeitsmenge in den Vorratsbehältern festzustellen, indem das Erreichen eines oberen Flüssigkeitsniveaus in den Vorratsbehältern ermittelt wird, und daß es darin besteht, das Vorhandensein einer festgelegten minimalen Flüssigkeitsmenge in den Vorratsbehältern festzustellen, indem das Erreichen des unteren Flüssigkeitsniveaus in den Vorratsbehältern ermittelt wird.

3. Verfahren nach einem der vorangehenden Ansprüche, dadurch gekennzeichnet, daß es außerdem darin besteht, zu überwachen, daß die maximale festgelegte Flüssigkeitsmenge gleichzeitig in beiden Vorratsbehältern erreicht wird, und daß die minimale festgelegte Flüssigkeitsmenge gleichzeitig in den beiden Vorratsbehältern erreicht wird.

4. Verfahren nach Anspruch 3, dadurch gekennzeichnet, daß es darin besteht, die in dem Venenvorratsbehälter (bzw. dem Arterienvorratsbehälter) vorhandene Flüssigkeitsmenge zu bestimmen, wenn die maximale festgesetzte Flüssigkeitsmenge in dem Arterienvorratsbehälter (bzw. dem Venenvorratsbehälter) erreicht wird, und die Luftmenge zu modifizieren, die im Innern zumindest eines Vorratsbehälters vorhanden ist, wenn die maximale vorbestimmte Flüssigkeitsmenge nicht gleichzeitig in den beiden Vorratsbehältern erreicht wird.

5. Verfahren nach einem der Ansprüche 3 oder 4, dadurch gekennzeichnet, daß es darin besteht, die Flüssigkeitsmenge zu bestimmen, die in dem Venenvorratsbehälter (bzw. dem Arterienvorratsbehälter) vorhanden ist, wenn die minimale vorbestimmte Flüssigkeitsmenge in dem Arterienvorratsbehälter (bzw. dem Venenvorratsbehälter) erreicht wird, und die Luftmenge zu modifizieren, die im Innern zumindest eines Vorratsbehälters vorhanden ist, wenn die minimale vorbestimmte Flüssigkeitsmenge nicht gleichzeitig in den beiden Vorratsbehältern erreicht wird.
